# EUROPEAN PATENT SPECIFICATION

(11) **EP 3 318 215 B1**
(45) Date of publication and mention of the grant of the patent: **13.05.2020**
(21) Application number: 16820889.0
(22) Date of filing: 30.06.2016
(51) Int. Cl.: A61C 5/50, A61C 13/30

(54) **DENTAL IMPLANTATION DEVICE FOR OBTURATING ROOT CANALS**
ZAHNIMPLANTATIONSVORRICHTUNG ZUR OBTURATION VON WURZELKANÄLEN
DISPOSITIF D'IMPLANTATION ODONTOLOGIQUE POUR OBTURER DES CANAUX RADICULAIRES

(30) Priority: 03.07.2015 ES 201530788 U
(43) Date of publication of application: 09.05.2018
(73) Proprietor: Institut Catala D'especialitats Odontologiques, S.L., 17310 Girona (ES)
(72) Inventor: OLLER PARDOS, Victor, 17310 Girona (ES); TORRES POLO, Francisco Javier, 17310 Girona (ES)
(74) Representative: Isern Patentes y Marcas S.L.
(86) International application number: PCT/ES2016/070489
(87) International publication number: WO 2017/005951

(56) References cited:
- WO-A1-93/19687
- WO-A2-2005/023132
- DE-A1- 3 620 527
- US-A- 4 832 602
- US-A- 5 051 093
- US-A1- 2003 148 247
- US-A1- 2005 003 328
- US-A1- 2010 150 985

## Description

### FIELD OF THE INVENTION

The dental implantation device of the present invention belongs to the technical field of dental implantation.

The object of the present invention is to provide a dental implantation device that incorporates significant innovations and advantages compared to the techniques of prior art.

More particularly, the invention proposes the development of a dental implantation device, which because of its particular properties, provides considerable advantages in the techniques of obturating root canals in teeth.

### BACKGROUND OF THE INVENTION

Various techniques for obturating root canals or endodontics in which a type of synthetic rubber referred to as gutta-percha is commonly used are known in the prior art.

The above-mentioned known techniques are based on the introduction into the orifice of the instrumented tooth, or the root canal (the orifice left after extraction from the root of the neurovascular bundle), of a rod of gutta-percha coated with a sealing cement so that the root canal, once it has been cleaned of bacteria, will be sealed and bacteriostatic.

Any bacteria remaining in the bone are thus prevented from re-entering and proliferating in the root canal, resulting in infection.

Other known techniques for facilitating the endodontic process include the use of gutta-percha plus cement, the use of silver or silver alloy points, and the use of plastic enclosed in gutta-percha.

In use of such techniques, however, it is often the case that a favorable and suitable seal of the apical or distal end of the root canal may not be achieved because of the difficulty of proper application of the technique or because of the morphology of the root canal itself.

In addition, said known techniques depend to a high degree on the expertise and practical proficiency of the professional, because if the point or rod used is longer than the root canal and extends outside of the root in its most apical area, the metal point remains lodged in the bone, and because it is not a perfectly biocompatible material, it causes undesirable reactions and problems such as rejection, and does not always adapt suitably to the anatomy of the root canal.

In other known techniques, in an effort to solve the above-described problem, plastic points covered entirely with gutta-percha are provided which, when heated in an oven provided for this purpose, take on a more fluid state, and when these points are introduced into the root canal, the walls of said canal are impregnated with said material.

Nevertheless, in said alternative techniques, the problem persists of the excessive length of the points or rods used at the bottom end of the root canal, with the result that gutta-percha may be introduced via the orifice at the bottom or end of the root canal and be lodged in the bone, which also makes these techniques dependent to a high degree on the expertise and practical proficiency of the professional.

Although gutta-percha is a synthetic plastic, this material is not perfectly biocompatible with the bone, and if the point or rod introduced is too long and protrudes through the end orifice at the bottom or end of the root canal, it will lodge in the bone, causing undesirable effects and reactions such as rejections.

Also, different patents are known in the current state of the art, and may be represented for example in the documents DE3620527A1, US2003148247A1 and US4832602A.

The document DE3620527A1 discloses a novel dental root pin for insertion and anchorage in a conically enlarged root canal of a tooth. The dental root pin is formed as a self-tapping pin having the shape of a truncated cone and being stable to bending for hermetically sealing, to interlocking insertion into the root canal. It is made of titanium or a titanium alloy which is physiologically acceptable. The outer surface of the dental root pin has lamellae to produce cutter-like roughening in order to permit fitting of the dental root pin in the root canal with the highest accuracy by filing or grinding to-and-fro movements of the pin. This is also the basis for the firm anchorage of the pin.
The document US2003148247A1 discloses, in connection with insertion of a post into a root canal, a simultaneous curing of the tangible sealing material aggregate. Gutta percha or other sealing material is removably attachable to the apical end of the post and heat or other energy is applied to the post, either before and/or after the post is inserted into the root canal of the tooth being treated, to plasticize and condense the tangible sealing material aggregate. The sealing material is removably integral with the post, or removably attached to the post. In other cases, the tangible sealing material aggregate is provided in a tapered tip and is attached to the bottom of the post without heat or other energy sources, or by condensation, and is therefore attached as an uncondensed single point fill. When placed in the root canal, the tangible sealing material aggregate tip is held in place by typical root canal cements, such as eugenol based cements, resin based cements and glass ionomer cements.

The document US4832602A discloses a stomatological implant in the form of selectively coated root canal pins, short pins, transfixion pins, replantation pins, of titanium, tantalum, niobium, zirconium and/or their alloys. The stomatological implant is used for long-term bacteria-proof closure of the root canal. Pursuant to the invention of US4832602A, up to three zones may be selectively put on the implant, meeting a variety of biological requirements.

The present invention contributes toward overcoming and solving the present problem, as its use makes it possible to provide considerable advantages in the techniques of obturating the root canals of teeth.

### SUMMARY OF THE INVENTION

The present invention was developed in order to provide a dental implantation device suitable for obturation of root canals in teeth that comprises an elongated rod having a lower section, a central section, and an upper section, is suited to be disposed in a tooth such that the lower section is inserted into the bottom or end of the root canal of the tooth, the central section is disposed along the length of the root canal, and the upper section is outside the root canal of the tooth, and wherein the lower section is composed of titanium, and the lower section has a geometry suitable for adaptation and/or incrustation in the area of the bottom or end of the root canal of the tooth, said geometry having a root canal sealing capacity, characterized in that the central section is composed of an alloy of titanium and nickel that is flexible for adaptation and positioning along the root canal, and the central section is coated with a coating of material that is flexible and biocompatible with the interior of the root canal.

In the dental implantation device, the coating of the central section is preferably composed of gutta-percha.

Alternatively, the coating of the central section of the dental implantation device is accompanied by an endodontic cement.

The lower section of the dental implantation device is preferably composed of titanium of grade V or above.

In other alternative embodiments, the lower section of the dental implantation device is composed of titanium of lower than grade V.

Alternatively, in the dental implantation device, the surface of the rod is treated by means of physicochemical microtreatments or nanotreatments.

Preferably, the lower section of the dental implantation device has a conical geometry.

The upper section of the rod of the dental implantation device preferably comprises a post-type prolongation, said post in turn having a slot suitable for the insertion of a pin present in an applicator, with the applicator therefore remaining fixed in relation to the post.

The present invention provides considerable advantages over the endodontic treatment techniques of the prior art, such as improved sealing, easy handling of the point-applicator/rod assembly, the superior biocompatibility of titanium of class V or above, ease of reconstruction of the treated tooth, easier selection of points for treatment of the root canal carried out/selected by a professional, and optimization of treatment time.

Other characteristics and advantages of the dental implantation device will be explained by describing a preferred but not exclusive embodiment illustrated as a non-limitative example in the accompanying drawings, which show the following:

### BRIEF DESCRIPTION OF THE FIGURES

Fig. 1 - A schematic view of a preferred embodiment of the dental implantation device of the present invention.
Fig. 2. - A schematic view of use in a tooth of a preferred embodiment of the dental implantation device of the present invention.
Fig. 3. - A schematic view of the same tooth shown in Fig. 2, but without using the dental implantation device of the present invention.

### DESCRIPTION OF A PREFERRED EMBODIMENT

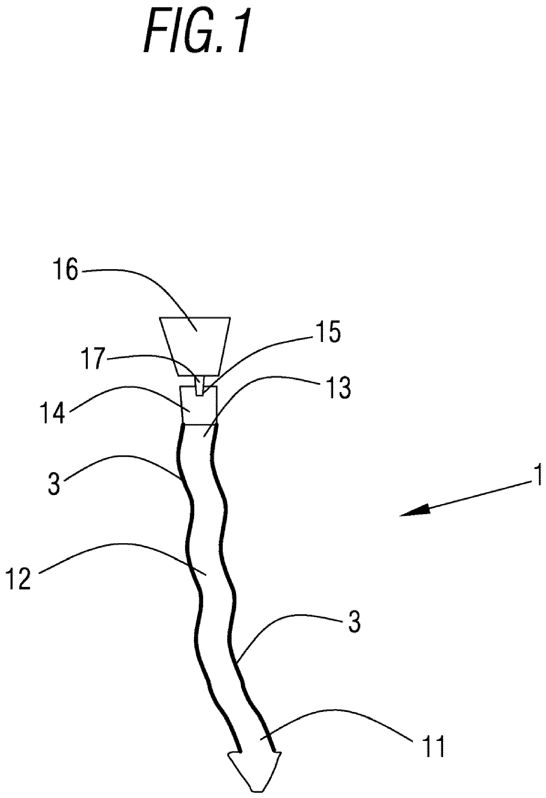

As shown schematically in Fig. 1, the dental implantation device of the invention comprises an elongated rod 1.

Said elongated rod 1 comprises a lower section 11, a central section 12, and an upper section 13.

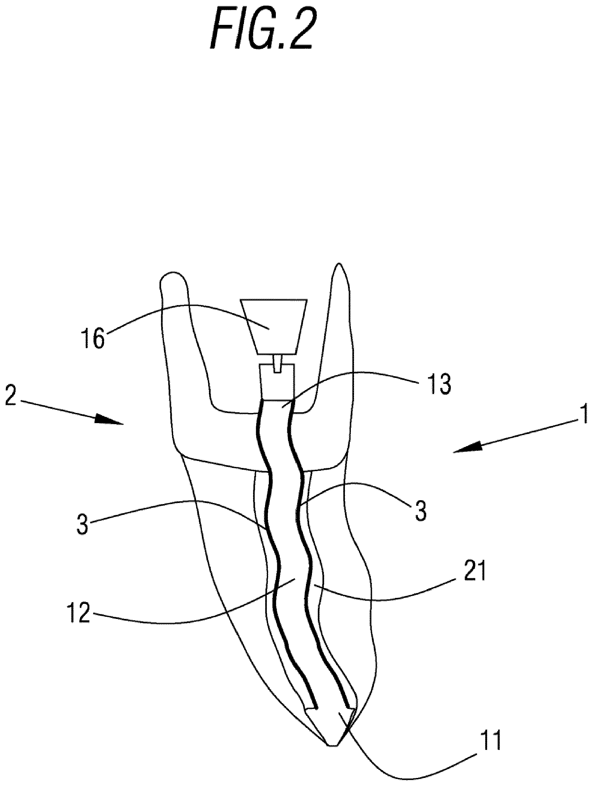

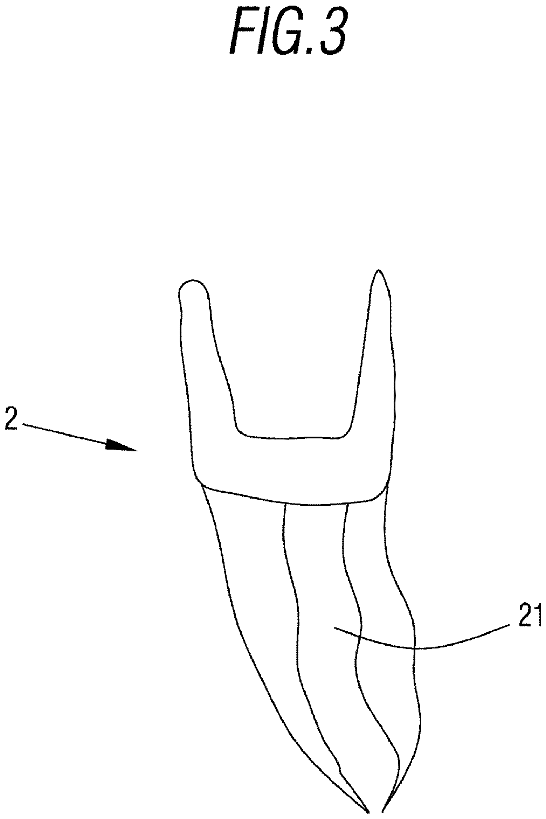

As shown schematically in Fig. 2, in use of the dental implantation device of the invention, the rod 1 is disposed and introduced into a tooth 2 such that the lower section 11 is inserted into the bottom or end of the root canal 21, the central section 12 is disposed along the length of the root canal 21, and the upper section 13 of the rod 1 is already outside the root canal 21 of the tooth 2. Fig. 3 shows a schematic view of the same tooth 2 for clarification purposes, but without the rod 1 introduced into it.

In this preferred embodiment, the lower section 11 has a conical or similar geometry so that it can be adapted and incrusted more effectively at the bottom of the root canal 21 of the tooth 2, thus ensuring that the root canal is sealed.

Because it will prospectively come into contact with the periapical bone that surrounds the bottom or end of the root canal 21, said lower section 11 is composed of a suitably osteointegrable material. For this purpose, it has a porous surface similar to that of the bone itself so that it can also be integrated into the bone surrounding the bottom of the root canal 21.

In this preferred embodiment, the lower section 11 of the rod 1 is composed of grade V titanium because of its favorable osteointegration properties.

In other preferred embodiments, the lower section 11 may be composed of titanium of higher than grade V, or even of titanium of lower than grade V, or another material suitable for this purpose having suitable osteointegration properties.

In other alternative embodiments, the surface of the rod 1 can be treated by means of physicochemical microtreatments or nanotreatments.

As shown schematically in Fig. 2, the central section 12 of the rod 1 is disposed along the length of the root canal 21 and must be composed of a material having sufficient flexibility to adapt itself to the shape and course of the said root canal 21.

In this preferred embodiment, the central section 12 is composed of an alloy of titanium and nickel (conventionally know as NiTi), because in addition to showing suitable flexibility, this alloy is also suitably biocompatible, with the result that it does not cause problems with rejection. In other preferred embodiments, the central section 12 may be composed of another material suitable for this purpose.

The upper section 13 of the rod 1 is already outside the root canal 21 of the tooth 2 and is sufficiently suitable for use and application in the dental implantation device.

In this preferred embodiment, as can be seen in Fig. 1, the upper section 13 of the rod 1 has a post-type prolongation 14. Said post 14 is used in reconstruction of the dental filling. The post 14 in turn can comprise a slot 15 for the insertion of an applicator 16. In this case, there is therefore no need for an additional metallic component or other material for the dental reconstruction, as the end of the applicator 16 itself fulfills this role.

For placement purposes, the applicator 16 comprises a suitable small pin 17 that is introduced into the slot 15 for radiological monitoring and selection of the suitable applicator 16.

In the dental implantation device of the present invention, the central section 12, which is suitably flexible for insertion and extension along the interior of the root canal 21, also comprises a coating 3 of a flexible material that is biocompatible with the interior of the root canal 21.

A material that is highly suitable for use in the coating 3 due to its properties of biocompatibility and flexibility is gutta-percha. In Figs. 1 and 2, said coating 3 is shown in bold for purposes of improved clarity.

In some preferred embodiments, before the rod 1 is introduced into the root canal 21, the coating 3 of gutta-percha can be heated so that it melts and takes on a fluid state, thus allowing it to penetrate more readily into the interstices of the root canal 21 and ensuring that it is properly sealed.

Because of its favorable properties of biocompatibility and flexibility, the gutta-percha of the coating 3 penetrates into the interstices of the inner walls of the root canal 21, thus ensuring that said root canal is properly and effectively sealed.

In other preferred embodiments, the coating 3 of gutta-percha can be accompanied by endodontic or similar cement in order thus to ensure the required sealing of the root canal 21.

The general geometry of the rod 1 can be adapted to that of the root canal 21 or to the instruments employed by the professional in using the dental implantation device of the invention.

The suitable and effective sealing of the interior of the root canal 21 of the tooth 2 provided by using the dental implantation device of the present invention prevents the establishment and proliferation of bacteria via the interior of said root canal and the resulting undesirable infections this would entail.

The details, shapes, dimensions, and other accessory elements, as well as the materials used in producing the dental implantation device of the present invention, may be replaced as desired by others that are technologically equivalent and do not depart from the scope defined by the claims attached below.

## Claims

1. A dental implantation device suitable for obturation of root canals in teeth that comprises an elongated rod (1) having a lower section (11), a central section (12), and an upper section (13), and is suited to be disposed in a tooth (2) such that the lower section (11) is adapted to be inserted into the bottom or end of a root canal (21) of the tooth (2), the central section (12) is adapted to be disposed along the length of the root canal (21), and the upper section (13) is adapted to be disposed outside the root canal (21) of the tooth (2), wherein the lower section (11) is composed of titanium, and the lower section (11) has a geometry suitable for adaptation and/or incrustation in the area of the bottom or end of the root canal (21) of the tooth (2), said geometry having a root canal sealing capacity, **characterized in that** the central section (12) is composed of an alloy of titanium and nickel that is flexible for adaptation and positioning along the root canal (21), and the central section (12) is coated with a coating (3) of material that is flexible and biocompatible with the interior of the root canal (21).

2. The dental implantation device as claimed in claim 1, **characterized in that** the coating (3) of the central section (12) is composed of gutta-percha.

3. The dental implantation device as claimed in claim 1 or 2, **characterized in that** the coating (3) of the central section (12) is accompanied by endodontic cement.

4. The dental implantation device as claimed in claim 1, **characterized in that** the lower section (11) is composed of titanium of grade V or above.

5. The dental implantation device as claimed in claim 1, **characterized in that** the lower section (11) is composed of titanium of lower than grade V.

6. The dental implantation device as claimed in claim 1, **characterized in that** the lower section (11) has a conical geometry.

7. The dental implantation device as claimed in claim 1, **characterized in that** the upper section (13) of the rod (1) comprises a post-type prolongation (14), wherein the post (14) in turn has a slot (15) suitable for the insertion of a pin (17) present in an applicator (16), said slot (15) and said pin (17) enabling the applicator (16) to remain fixed in relation to the post (14).

## Patentansprüche

1. Zahnimplantationsvorrichtung, die für die Obturation von Wurzelkanälen in Zähnen geeignet ist, die einen länglichen Stab (1) mit einem unteren Abschnitt (11), einem mittleren Abschnitt (12) und einem oberen Abschnitt (13) umfasst und geeignet ist, in einem Zahn (2) angeordnet zu werden, sodass der untere Abschnitt (11) dazu angepasst ist, in den Boden oder das Ende eines Wurzelkanals (21) des Zahns (2) eingesetzt zu werden, der mittlere Abschnitt (12) dazu angepasst ist, entlang der Länge des Wurzelkanals (21) angeordnet zu werden, und der obere Abschnitt (13) dazu angepasst ist, außerhalb des Wurzelkanals (21) des Zahns (2) angeordnet zu werden, wobei der untere Abschnitt (11) aus Titan besteht und der untere Abschnitt (11) eine Geometrie aufweist, die zur Anpassung und/oder Inkrustation im Bereich des Bodens oder Endes des Wurzelkanals (21) des Zahns (2) geeignet ist, wobei die Geometrie eine Fähigkeit zur Wurzelkanalversiegelung aufweist, **dadurch gekennzeichnet, dass** der mittlere Abschnitt (12) aus einer Legierung aus Titan und Nickel besteht, die für die Anpassung und die Positionierung entlang des Wurzelkanals (21) flexibel ist, und dass der mittlere Abschnitt (12) mit einer Beschichtung (3) aus einem Material beschichtet ist, das flexibel und mit dem Inneren des Wurzelkanals (21) biokompatibel ist.

2. Zahnimplantationsvorrichtung nach Anspruch 1, **dadurch gekennzeichnet, dass** die Beschichtung (3) des mittleren Abschnitts (12) aus Guttapercha besteht.

3. Zahnimplantationsvorrichtung nach Anspruch 1 oder 2, **dadurch gekennzeichnet, dass** die Beschichtung (3) des mittleren Abschnitts (12) mit endodontischem Zement einhergeht.

4. Zahnimplantationsvorrichtung nach Anspruch 1, **dadurch gekennzeichnet, dass** der untere Abschnitt (11) aus Titan Grade V oder höher besteht.

5. Zahnimplantationsvorrichtung nach Anspruch 1, **dadurch gekennzeichnet, dass** der untere Abschnitt (11) aus Titan eines niedrigeren als Grade V besteht.

6. Zahnimplantationsvorrichtung nach Anspruch 1, **dadurch gekennzeichnet, dass** der untere Abschnitt (11) eine konische Geometrie aufweist.

7. Zahnimplantationsvorrichtung nach Anspruch 1, **dadurch gekennzeichnet, dass** der obere Abschnitt (13) des Stabs (1) eine pfostenartige Verlängerung (14) umfasst, wobei der Pfosten (14) wiederum einen Schlitz (15) aufweist, der zum Einsetzen eines Stiftes (17) geeignet ist, der in einem Applikator (16) vorhanden ist, wobei der Schlitz (15) und der Stift (17) es dem Applikator (16) ermöglichen, in Bezug auf den Pfosten (14) fixiert zu bleiben.

## Revendications

1. Dispositif d'implantation dentaire approprié à l'obturation de canaux radiculaires dans les dents qui comprend une tige allongée (1) ayant une section inférieure (11), une section centrale (12), et une section supérieure (13), et est approprié pour être disposé dans une dent (2) de sorte que la section inférieure (11) est adaptée pour être insérée dans le fond ou l'extrémité d'un canal radiculaire (21) de la dent (2), la section centrale (12) est adaptée pour être disposée le long du canal radiculaire (21), et la section supérieure (13) est adaptée pour être disposée à l'extérieur du canal radiculaire (21) de la dent (2), dans lequel la section inférieure (11) est composée de titane, et la section inférieure (11) a une géométrie appropriée à l'adaptation et/ou l'incrustation dans la zone du fond ou de l'extrémité du canal radiculaire (21) de la dent (2), ladite géométrie ayant une capacité d'étanchéité de canal radiculaire, **caractérisé en ce que** la section centrale (12) est composée d'un alliage de titane et de nickel qui est flexible pour l'adaptation et le positionnement le long du canal radiculaire (21), et la section centrale (12) est revêtue d'un revêtement (3) de matériau qui est flexible et biocompatible avec l'intérieur du canal radiculaire (21).

2. Dispositif d'implantation dentaire tel que revendiqué dans la revendication 1, **caractérisé en ce que** le revêtement (3) de la section centrale (12) est composé de gutta-percha.

3. Dispositif d'implantation dentaire tel que revendiqué dans la revendication 1 ou 2, **caractérisé en ce que** le revêtement (3) de la section centrale (12) est accompagné de ciment endodontique.

4. Dispositif d'implantation dentaire tel que revendiqué dans la revendication 1, **caractérisé en ce que** la section inférieure (11) est composée de titane de grade V ou supérieur.

5. Dispositif d'implantation dentaire tel que revendiqué dans la revendication 1, **caractérisé en ce que** la section inférieure (11) est composée de titane de grade inférieur à V.

6. Dispositif d'implantation dentaire tel que revendiqué dans la revendication 1, **caractérisé en ce que** la section inférieure (11) a une géométrie conique.

7. Dispositif d'implantation dentaire tel que revendiqué dans la revendication 1, **caractérisé en ce que** la section supérieure (13) de la tige (1) comprend un prolongement de type tenon (14), dans lequel le tenon (14) a à son tour une fente (15) appropriée à l'insertion d'une broche (17) présente dans un applicateur (16), ladite fente (15) et ladite broche (17) permettant à l'applicateur (16) de rester fixe par rapport au tenon (14).
